# EUROPEAN PATENT SPECIFICATION

(11) **EP 2 405 870 B1**
(45) Date of publication and mention of the grant of the patent: **03.06.2015**
(21) Application number: 10709162.1
(22) Date of filing: 01.03.2010
(51) Int. Cl.: A61F 5/44

(54) **BODY FLUID COLLECTION POUCH WITH ANTI-RETURN VALVE**
BEUTEL ZUR SAMMLUNG VON KÖRPERFLÜSSIGKEITEN MIT RÜCKFLUSS VERHINDERNDEM VENTIL
POCHE COLLECTRICE DE LIQUIDES ORGANIQUES À CLAPET ANTI-RETOUR

(30) Priority: 06.03.2009 FR 0951409
(43) Date of publication of application: 18.01.2012
(73) Proprietor: B. Braun Medical SAS, 92100 Boulogne Billancourt (FR)
(72) Inventor: BORDEAU, Jérôme, F-61110 Le Condeau (FR)
(74) Representative: Mahler, Peter
(86) International application number: PCT/EP2010/001247
(87) International publication number: WO 2010/099916

(56) References cited:
- EP-A- 0 248 657
- EP-A- 1 825 838
- GB-A- 2 202 032
- GB-A- 2 394 668

## Description

The present invention relates to a fluid collection pouch. More specifically, the present invention allows collecting body fluids such as, for example, blood or urine. In addition to this particularity, the invention also makes it possible to store solutions used for infusion, for washing or rinsing wounds or medical instruments.

The use of body fluid collection pouches, as described e.g. in patent application EP 1 825 838, is well known. This document describes a collection pouch onto which an anti-return valve is fixed whose principal function is to prevent any reflux whatsoever of the liquid contained within said collection pouch. By way of illustration, we may cite the draining and collection of urine during a urinary catheterization procedure, also known as bladder catheterization. During the implementation of such a bladder catheterization procedure, the device often comprises not only a urinary catheter, but also a collection pouch with an anti-reflux valve. Such a valve prevents the return of fluid, i.e. of the urine collected and stored in the collection pouch, into the urinary catheter and eventually the bladder.

However, due to the geometric complexity and the number of components of the devices according to the state of the art, manufacturing such devices requires advanced and specific know-how as well as expensive workforce and manufacturing equipment. More specifically, the collection pouches according to the state of the art comprise in particular a pouch and an anti-reflux valve, the latter being constituted by generally more than one piece which must be assembled together.

On this background, the invention aims to offer a device which overcomes the aforementioned problems and offers the medical profession a device with few parts, cheap and perfectly suited to the practice and the requirements of f.i. vesical catheterization. The collection pouch according to the invention makes it possible in particular to collect and retain the liquid, even when a pressure or traction stress is applied to it.

To this end, the invention relates to a collection pouch for body fluids which is provided with an anti-reflux valve, said collection pouch being constituted of two sheets of plastic material, superposed and welded along their periphery, said collection pouch comprising:
- a collection compartment;
- an admission orifice; and
- an admission channel located between said admission orifice and the entrance of said collection compartment, said admission channel being delineated by welds which constitute said anti-reflux valve,
said collection pouch being **characterised in that:**
- said admission orifice and said entrance to said collection compartment are offset, in particular axially, in relation to one another,
- said welds of said admission channel define the internal limits of said collection compartment of the collection pouch,
- said admission channel opens into said collection compartment distant from the lateral walls of the collection pouch.

In addition to the principal characteristics mentioned in the preceding paragraph, the device according to the invention may have one or more of the supplementary characteristics below, considered individually or in any of the technically possible combinations:
- said admission channel extends into said collection compartment in such a way as to form, on at least one side of said entrance, a collection sub-compartment;
- said admission channel is configured in such a way that a straight line joining the admission orifice and any point on the periphery of the collection compartment crosses at least one of said welds of said admission channel;
- one of said welds is essentially parallel to the axis of said admission orifice;
- said entry into said collection compartment is of a width between 14 and 20 mm;
- said admission channel is of a width greater than or equal to 14 mm and in particular greater than or equal to 20 mm;
- said welds, at the points of concentration of stresses, have a minimum width of 2 mm, in particular between 2 and 8 mm, preferably between 4 and 6 mm;
- the borders of said welds are configured radially, i.e. they do not form any angles at which the pressure stresses might concentrate when the pouch is filled;
- the width of said sub-compartment is greater than or equal to 20 mm, in particular between 20 and 40 mm, preferably between 25 and 35 mm, and the height of at least one sub-compartment is greater than or equal to 10 mm, in particular between 10 and 30 mm, preferably between 15 and 25 mm;
- the lower extremities of said welds are aligned.

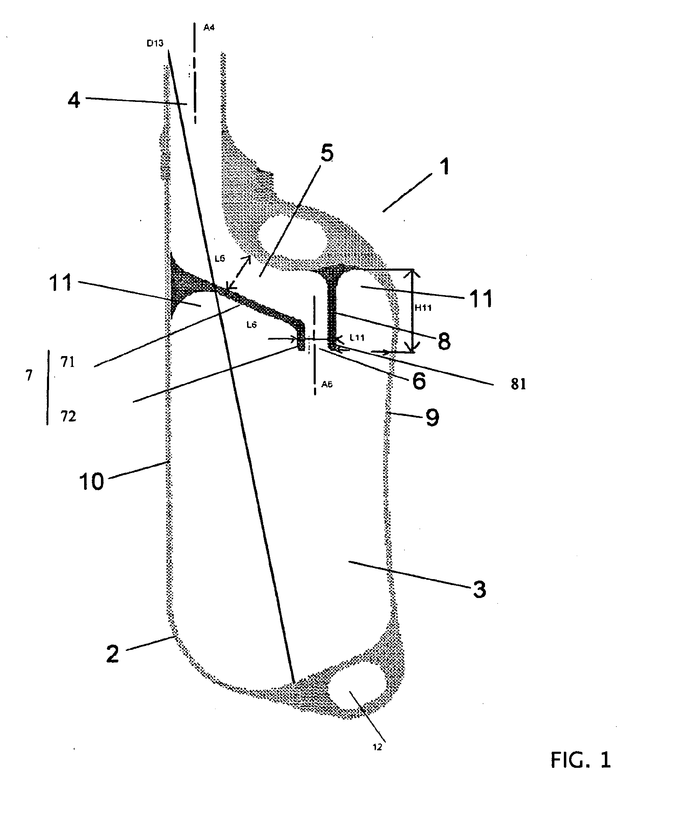

Other characteristics and advantages of the invention will emerge clearly from the description given below, which is by way of indication and in no way limiting, of the modes of realisation making reference to figure 1 attached, on which is represented an example of a device according to the invention.

For the sake of clarity, only the elements useful for the understanding of the invention have been shown, schematically and not to scale.

**Figure 1** illustrates a collection pouch **1** making it possible to collect any type of fluid, and in particular urine. This pouch comprises, notably:
- an admission orifice **4;**
- an admission channel **5;**
- an entrance 6 to a collection compartment **3;**
- two welds **7** and **8;**
- two sub-compartments **11;**
- an eyelet **12;**
- two lateral welds **9** and **10** of the collection compartment **3,** which form the lateral edges of the collection compartment **3.**

Moreover, a straight line **D13** is shown on figure 1.

The collection pouch **1** illustrated in figure 1 is formed of two sheets made, for example, of plastic material. During the assembly of the collection pouch **1,** these sheets are superposed and welded along the whole of their periphery **2** such as to form a supple pouch. In other words, these two sheets by themselves form the bodily fluid collection pouch 1.

The welds **7** and **8** are realized between the two sheets of the collection pouch **1.** These welds **7** and **8** may be, for example, heat-welds.

The weld **8** is essentially vertical (parallel to the axis A4) and comprises a lower extremity **81.**

The weld 7 comprises a first oblique part 71 and a second essentially vertical part **72** (parallel to A4).

Advantageously, the lower extremities **72** and **81** of the welds **7** and **8** form the entrance **6** to the collection compartment **3** and finish at an essentially identical height.

Moreover, the two welds 7 and 8 form, on the one hand, an anti-reflux valve, the functioning of which is described below, and on the other hand, the lower part of the admission **channel 5.**

The anti-reflux valve, that is, the assembly formed by the welds **7** and **8,** is offset axially from the admission orifice **4.**

The first sub-compartment **11** is formed by the weld **10** and the weld **7.**

The second sub-compartment **11** is formed by the weld **8** and the weld **9.**

Note that the welds **9, 10** and the two welds **7** and **8** delimit the collection compartment **3.**

Thus, when a liquid is introduced via the admission orifice **4,** it runs down the admission channel **5** formed by the two welds **7, 8** and the two sheets. Consequently, the collection compartment **3** is filled via the intermediary of the admission channel **5.**

In order to ensure uniform and regular flow, the width **L5** of the admission channel **5** is preferably greater than or equal to 14 mm and in particular greater than or equal to 20 mm. Likewise, the width **L6** of the anti-reflux valve is, preferably, between **14** and 20 mm.

When the fluids contained in the collection compartment **3** fill the sub-compartments **11,** for example when the user of the pouch turns it upside-down, the anti-reflux valve closes and prevents any reflux of fluid through the admission **channel 5.** More specifically, when the sub-compartments **11** fill with fluid, the ensuing hydrostatic pressure is the same at all points of the walls and welds of these sub-compartments. The result of this hydrostatic pressure, however, is a specific configuration of tensions in the walls of the sub-compartments which tend to separate the welds **72** and **82** of the entrance **6** of said anti-reflux valve from one another and thereby to close this entrance **6,** this closure becoming all the more hermetic as the pressure of fluid in the sub-compartments **11** increases. This closure prevents any reflux of liquid towards the exterior of the collection compartment **3,** even if the collection pouch 1 is turned upside-down or subjected to any compression and/or traction stress.

In order to ensure that the anti-reflux valve of the collection pouch **1** is perfectly tight, the width **L11** of the sub-compartments **11** measures between 20 and 40 mm, preferably between 25 and 35 mm, and the height **H11** of said sub-compartments measures between 10 and 30 mm, preferably between **15** and 25 mm.

The volume formed by the width **L11** and the height **H11** corresponds to the minimum volume necessary to ensure the tightness of the anti-reflux valve.

Moreover, as shown in figure 1, the admission orifice **4,** formed by the upper extremity of the admission channel **5,** is offset axially from the entrance **6** to the collection compartment **3** formed by the lower extremity of the admission channel **5.** This axial offsetting of the **A4** and **A6** axes makes it possible to maintain the tightness of the anti-reflux valve when the collection compartment **3** is filled and when a traction stress is exerted on the collection pouch **1** at the level of the eyelet **12** and of the admission orifice **4.**

More specifically, this axial offset may, for example, be modelled by the straight line **D13.** This latter passes through the admission orifice **4** to join any point of the perimeter **2** of the two lateral welds **9** and **10** of the collection compartment **3,** and must necessarily cut across one of the welds **7** or **8.** This layout makes it possible to ensure that the anti-reflux valve remains tight, even if a traction or compression stress is applied to it.

Moreover, the weld **8** is essentially parallel to the axis **A4** of the admission orifice **4.** This particularity makes it possible to prevent the anti-reflux valve from opening when a traction effort is applied to the collection pouch **1.**

In addition, the welds **7** and **8** are on the one hand configured radially, that is, they do not form any angles in which the pressure stresses might concentrate when the pouch is filled, and, on the other hand, have a width between 2 and 8 mm, preferably between **4** and **6** mm. This width is particularly important at the points of concentration of the stresses and avoids that the welds **7** and **8** are weakened and disrupt.

In short, the collection pouch **1** has in particular the advantage of offering a device comprising an anti-reflux valve that does not require any additional part or element, thus allowing the collection pouch **1** and the anti-reflux valve to be manufactured by a specific and ingenious combination of one or more welds of the two sheets of the body fluid collection pouch **1.**

The collection pouch **1** according to the invention is tight when it is manipulated, and more specifically when a traction is applied to its two extremities.

The present invention has a direct application in the context of medical care, and in particular the field of managing urinary incontinence, whether in a hospital environment or in the community.

The invention is described in the above by way of example. It is understood that the person skilled in the art is able to carry out different variants of the collection pouch, in particular relating to the positioning or the layout of the welds that form the anti-reflux valve, without departing from the invention as defined in the claims.

## Claims

1. Collection pouch (1) for body fluids provided with an anti-reflux valve, said collection pouch (1) being constituted of two sheets of plastic material superposed and welded along the whole of their periphery (2), said collection pouch (1) comprising:
- a collection compartment (3);
- an admission orifice (4) and;
- an admission channel (5) located between said admission orifice (4) and an entrance (6) to said collection compartment (3), said admission channel (5) being defined by welds (7, 8) forming the anti-reflux valve,
said collection pouch (1) being **characterised in that:**
- said admission orifice (4) and said entrance (6) to said collection compartment (3) are offset in relation to one another,
- said welds (7, 8) of said admission channel (5) define the internal limits of said collection compartment (3) of said collection pouch (1),
- said admission channel (5) opens into said collection compartment (3) distant from the lateral walls (9, 10) of the collection pouch (1).

2. Collection pouch (1) according to claim 1, **characterised in that** said admission channel (5) extends into said collection compartment (3) such as to form, on at least one side of said entrance (6), a collection sub-compartment (11).

3. Collection pouch (1) according to the preceding claim, **characterised in that** the width (L11) of said sub-compartment (11) is greater than or equal to 20 mm and in particular between 20 and 40 mm, preferably between 25 and 35 mm, and the height (H11) of said at least one sub-compartment (11) is greater than or equal to 10 mm and in particular between 10 mm and 30 mm, preferably between 15 and 25 mm.

4. Collection pouch (1) according to one of the preceding claims, **characterised in that** said admission channel (5) is configured in such a way that a straight line (D13) joining said admission orifice (4) and any point on the perimeter (2) of said collection compartment (3) crosses at least one of said welds (7, 8) of said admission channel (5).

5. Collection pouch (1) according to one of the preceding claims, **characterised in that** one of said welds (7, 8) is essentially parallel to the axis (A4) of said admission orifice (4).

6. Collection pouch (1) according to one of the preceding claims, **characterised in that** said entrance (6) to said collection compartment (3) has a width (L3) between 14 and 20 mm.

7. Collection pouch (1) according to one of the preceding claims, **characterised in that** said admission channel (5) has a width (L5) greater than or equal to 14 mm and in particular greater than or equal to 20 mm.

8. Collection pouch (1) according to one of the preceding claims, **characterised in that** the lower extremities of said welds (7, 8) are aligned.

9. Collection pouch (1) according to one of the preceding claims, **characterised in that** said welds (7, 8) have, at the points of concentration of stresses, a minimum width of 2 mm and in particular between 2 and 8 mm, preferably between 4 and 6 mm.

10. Collection pouch (1) according to one of the preceding claims, **characterised in that** said welds (7, 8) are configured radially.

## Patentansprüche

1. Sammelbeutel (1) zum Sammeln von Körperflüssigkeiten mit einem den Rückfluss verhindernden Ventil, der aus zwei übereinanderliegenden und über ihren gesamten Umfang (2) verschweißten Lagen eines Kunststoffmaterials besteht, umfassend:
- eine Sammelkammer (3);
- eine Einlassöffnung (4) und;
- einen zwischen der Einlassöffnung (4) und einem Eingang (6) zur Sammelkammer (3) angeordneten Einlasskanal (5), der durch Nähte (7, 8) begrenzt ist, welche das den Rückfluss verhinderne Ventil bilden,
wobei der Beutel (1) **dadurch gekennzeichnet ist, dass:**
- die Einlassöffnung (4) und der Eingang (6) zur Sammelkammer (3) zueinander versetzt sind,
- die Nähte (7, 8) des Einlasskanals (5) die inneren Grenzen der Sammelkammer (3) des Beutels (1) bilden,
- der Einlasskanal (5) beabstandet von den Seitenwänden (9, 10) des Sammelbeutels (1) in die Sammelkammer (3) mündet.

2. Sammelbeutel (1) nach Anspruch 1, **dadurch gekennzeichnet, dass** der Einlasskanal (5) derart in die Sammelkammer (3) mündet, dass zumindest auf einer Seite des Eingangs (6) ein Unterabschnitt (11) entsteht.

3. Sammelbeutel (1) nach dem vorangehenden Anspruch, **dadurch gekennzeichnet, dass** die Breite (L11) des Unterabschnitts (11) größer oder gleich 20 mm ist, insbesondere zwischen 20 und 40 mm, vorzugsweise zwischen 25 und 35 mm, und die Höhe (H11) des mindestens einen Unterabschnitts (11) größer oder gleich 10 mm ist, insbesondere zwischen 10 mm und 30 mm, vorzugsweise zwischen 15 und 25 mm.

4. Sammelbeutel (1) nach einem der vorhergehenden Ansprüche, **dadurch gekennzeichnet, dass** der Einlasskanal (5) so gestaltet ist, dass eine gerade Linie (D13), die die Einlassöffnung (4) und einen beliebigen Punkt des Umfangs (2) der Sammelkammer (3) miteinander verbindet, mindestens eine der Nähte (7, 8) des Einlasskanals (5) schneidet.

5. Sammelbeutel (1) nach einem der vorhergehenden Ansprüche, **dadurch gekennzeichnet, dass** eine der Nähte (7, 8) im Wesentlichen parallel zur Achse (A4) der Einlassöffnung (4) verläuft.

6. Sammelbeutel (1) nach einem der vorhergehenden Ansprüche, **dadurch gekennzeichnet, dass** der Eingang (6) zur Sammelkammer (3) eine Breite (L3) zwischen 14 und 20 mm aufweist.

7. Sammelbeutel (1) nach einem der vorhergehenden Ansprüche, **dadurch gekennzeichnet, dass** der Einlasskanal (5) eine Breite (L5) größer oder gleich 14 mm aufweist, insbesondere größer oder gleich 20 mm.

8. Sammelbeutel (1) nach einem der vorhergehenden Ansprüche, **dadurch gekennzeichnet, dass** die unteren Endpunkte der Nähte (7, 8) aufeinander ausgerichtet sind.

9. Sammelbeutel (1) nach einem der vorhergehenden Ansprüche, **dadurch gekennzeichnet, dass** die Nähte (7, 8) an den Punkten mit der höchsten Spannungsbelastung eine Breite von mindestens 2 mm und insbesondere zwischen 2 und 8 mm aufweisen, vorzugsweise zwischen 4 und 6 mm.

10. Sammelbeutel (1) nach einem der vorhergehenden Ansprüche, **dadurch gekennzeichnet, dass** die Nähte (7, 8) strahlenförmig angeordnet sind.

## Revendications

1. Poche de recueil (1) de fluides corporels munie d'une valve anti-reflux, ladite poche de recueil (1) étant constituée de deux feuilles en matière plastique superposées et soudées le long de leur pourtour (2), ladite poche de recueil (1) comportant :
- un compartiment de recueil (3) ;
- un orifice d'admission (4) et ;
- un canal d'admission (5) situé entre ledit orifice d'admission (4) et une entrée (6) dudit compartiment de recueil (3), ledit canal d'admission (5) étant défini par des soudures (7, 8) formant la valve anti-reflux,
ladite poche de recueil (1) étant **caractérisée en ce que :**
- ledit orifice d'admission (4) et ladite entrée (6) dudit compartiment de recueil (3) sont décalés l'une par rapport à l'autre,
- lesdites soudures (7, 8) dudit canal d'admission (5) définissent les limites internes dudit compartiment de recueil (3) de ladite poche de recueil (1),
- ledit canal d'admission (5) débouche dans ledit compartiment de recueil (3) espacé des parois latérales (9, 10) de la poche de recueil (1).

2. Poche de recueil (1) selon la revendication 1, **caractérisée en ce que** ledit canal d'admission (5) s'avance dans ledit compartiment de recueil (3) de manière à former au moins d'un côté de ladite entrée (6), un sous-compartiment de recueil (11).

3. Poche de recueil (1) selon la revendication précédente **caractérisée en ce que** la largeur (L11) dudit sous-compartiment (11) est supérieure ou égale à 20 mm et notamment comprise entre 20 et 40 mm, de préférence entre 25 et 35 mm, et la hauteur (H11) dudit au moins un sous-compartiment (11) est supérieure ou égale à 10 mm et notamment comprise entre 10 et 30 mm, de préférence entre 15 et 25 mm.

4. Poche de recueil (1) selon l'une des revendications précédentes, **caractérisée en ce que** ledit canal d'admission (5) est configuré de manière à ce qu'une droite (D13) reliant ledit orifice d'admission (4) et un point quelconque du pourtour (2) dudit compartiment de recueil (3) traverse au moins une desdites soudures (7, 8) dudit canal d'admission (5).

5. Poche de recueil (1) selon l'une des revendications précédentes, **caractérisée en ce qu'**une desdites soudures (7, 8) est sensiblement parallèle à l'axe (A4) dudit orifice d'admission (4).

6. Poche de recueil (1) selon l'une des revendications précédentes **caractérisée en ce que** ladite entrée (6) dudit compartiment de recueil (3) possède une largeur (L3) comprise entre 14 et 20 mm.

7. Poche de recueil (1) selon l'une des revendications précédentes **caractérisée en ce que** ledit canal d'admission (5) possède une largeur (L5) supérieure ou égale à 14 mm et notamment supérieure ou égale à 20 mm.

8. Poche de recueil (1) selon l'une des revendications précédentes **caractérisé en ce que** les extrémités inférieures desdites soudures (7, 8) sont alignées.

9. Poche de recueil (1) selon l'une des revendications précédentes **caractérisée en ce que** lesdites soudures (7, 8) possèdent, aux points de concentration des contraintes, une épaisseur minimum de 2 mm et notamment comprise entre 2 et 8 mm, de préférence entre 4 et 6 mm.

10. Poche de recueil (1) selon l'une des revendications précédentes **caractérisée en ce que** lesdites soudures (7, 8) sont rayonnées.
